# EUROPEAN PATENT APPLICATION

(11) **EP 0 538 534 A1**
(43) Date of publication of application: **28.04.1993**
(21) Application number: 91402864.2
(22) Date of filing: 25.10.1991
(51) Int. Cl.: C07D 471/14, C07D 491/22, A61K 31/47

(54) **Improved process for chemical reactions on camptothecin or analogs thereof**

(71) Applicant: RESEARCH TRIANGLE INSTITUTE, Research Triangle Park, North Carolina 27709-2194 (US)
(72) Inventor: Wall, Monroe E., Chapel Hill, North Carolina 27514 (US); Wani, Mansukh C., Durham, North Carolina 27707 (US); Tele, Chhagan A., Durham, North Carolina 27707 (US)
(74) Representative: Polus, Camille

(57) **Abstract**

A process for preparing a reduced camptothecin derivative in which a camptothecin substrate bearing a reducible substituent is hydrolyzed under alkaline conditions to open the lactone E ring and form a carboxylate salt. The carboxylate salt is reduced to form a reduced carboxylate salt which is then contacted with acid to reform the lactone E ring. This procedure results in substantially increased yields of camptothecin derivatives.

## Description

### BACKGROUND OF THE INVENTION

### Field of the Invention:

The invention is directed to an improved process for conducting reactions on compounds having the camptothecin structure. More specifically, the invention is directed to a process for easily conducting chemical reactions in aqueous medium with improved yields of the camptothecin compound.

### Discussion of the Background:

Camptothecin is a pentacyclic alkaloid initially isolated from the wood and bark of Camptotheca acuminata by Wall et al (M. E. Wall, M. C. Wani, C. E. Cook, K. H. Palmer, A. T. McPhail and G. A. Sim, J. Am. Chem. Soc., 94: 388 (1966)). The parent camptothecin is highly biologically active and displays strong inhibitory activity toward the biosynthesis of nucleic acids. Camptothecin exhibits potent anti-tumor activity against experimentally transplanted carcinoma such as Leukemia L-1210 in mice or Walker 256 tumor in rats.

The potent anti-tumor activity of camptothecin has generated considerable synthetic interest in preparing analogs of camptothecin. Methods of preparing camptothecin analogs and various camptothecin analogs are disclosed, for example, in U.S. 4,894,456, 4,604,463, 4,545,880, 4,473,690 and 4,431,098 as well as the references discussed in these U.S. Patents.

Camptothecin and many of its analogs have very limited solubility not only in water but in most organic solvents. As a consequence, once the pentacyclic ring is formed, it is difficult to conduct further reactions on a camptothecin molecule. The low solubility of camptothecin and its analogs requires the use of large volumes of solvents. These large solvent volumes give rise to problems relating to solvent disposal, solvent recycling, solvent cost and the loss of product yield due to losses of compound during handling. Purification of the product camptothecin or camptothecin analog as well as intermediates in a synthetic sequence by crystallization or by chromatography is hindered and rendered costly and/or impractical due to the inordinately large volumes of solvent required.

A need continues to exist, therefore, for new and improved methods of preparing and purifying camptothecin and camptothecin analogs which eliminate the use of large solvent volumes required by prior art processes.

### SUMMARY OF THE INVENTION

Accordingly, one object of the present invention is to provide a process for preparing camptothecin and camptothecin analogs which does not suffer from the deficiencies of prior art processes noted above.

A further object is to provide a process for preparing camptothecin and its analogs which allows one to obtain the product camptothecin compound in high yield without the need for barge volumes of expensive solvents.

These and other objects which will become apparent from the following specification have been achieved by the present process in which a camptothecin compound, in which the A, B, C or D ring is substituted with a substituent which is capable of being reduced under alkaline or neutral conditions, is hydrolyzed to form a water soluble substituted carboxylate salt. The reducible substituent on the carboxylate salt is then reduced in neutral or alkaline reaction media to form a reduced camptothecin salt. The water soluble reduced camptothecin derivative may then be purified by column chromatography followed by crystallization. It is then contacted with an acid to reform the lactone E ring and thereby form a new modified camptothecin compound.

### DETAILED DESCRIPTION OF THE PREFERRED EMBODIMENTS

The camptothecin compound which serves as the substrate for the process of the present invention has the well-known camptothecin skeletal structure containing rings A-E and additionally containing at least one substituent (R) on one of the A, B, C or D rings which is capable of being reduced under alkaline or neutral conditions. The substrate may be camptothecin itself in which the substituent is a hydrogen atom on the A-D rings. Alternatively, the substrate may be a camptothecin compound bearing a non-hydrogen substituent which is capable of being reduced under alkaline or neutral conditions.

The structure shown below and all strutures in scheme 1 below represent any and all isomers of the camptothecin compound having the structure indicated. For example, the structures represent both the 20(S) and 20(R) enantiomers of camptothecin compounds which occur due to the different configurations possible at the 20-position in the E ring of the camptothecin compounds. Also included within the scope of the invention are all isomers possible due to the structure and/or location of the R substituent. 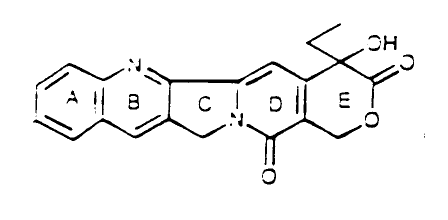
The term "reduction" used in its broad chemical sense means any reaction, in which electrons are transferred to the substrate from a reducing agent. A substituent which is capable of being reduced, is a substituent in which electrons can be transferred to the substituent either from a reducing agent or from other portions of the camptothecin skeletal structure.

Substituents (R) which may be reduced include nitro, cyano, azido, alkenyl, alkynyl, formyl, halides (Cl, Br, I, F), nitroso, etc. Preferred alkenyl, alkynyl and acyl groups have 2-10 carbon atoms. The forgoing examples are illustrative only, and it is to be understood that any substituent which can be reduced under neutral or alkaline conditions using known reduction methods can be present as the substituent on the camptothecin substrate for the present process. Of course, more than one substituent (R) may be present on the camptothecin substrate. The number of substituents is preferably one, but may be two, three or more and is dependent only on the availability of the desired camptothecin substrate.

The camptothecin substrate may also bear additional substituents which are non-reactive in the reducing step. Examples of non-reactive substituents include alkyl, hydroxyalkyl, alkylamino, aminoalkyl, amino, aminodialkyl, alkylether, etc. Preferred non-reactive groups are those containing up to 10 carbon atoms, preferably up to 6 carbon atoms. Particularly preferred non-reactive groups are the methylenedioxy (-OCH₂O-) and ethylenedioxy (-OCH₂CH₂O-) groups which occupy two positions on the camptothecin substrate, typically two positions on the A-ring of the camptothecin structure. When a methylenedioxy or ethylenedioxy group is present, the reducible substituent R may be present at one or more of the remaining available substituent positions on the camptothecin skeletal structure. For example, typical substrates include 9-nitro-10,11-methylenedioxycamptothecin, 12-nitro-10,11-methylenedioxycamptothecin, 7-C₁₋₁₀ alkyl-9-nitro-10,11-methylenedioxycamptothecin and the corresponding 10,11-ethylenedioxycamptothecin compounds. The 9-nitro and 12-nitro derivatives of methylenedioxycamptothecin and ethylenedioxycamptothecin are available by conventional nitration of the methylenedioxycamptothecin or ethylenedioxycamptothecin using nitric/sulfuric acid.

In the process of the present invention, the substituted camptothecin is first hydrolyzed under alkaline conditions to give a substituted carboxylate salt in which the E ring has been hydrolyzed to the corresponding carboxylate and hydroxyl groups. The hydrolysis reaction is preferably conducted in aqueous solution using a suitable hydroxide base. Preferred hydroxide bases are metal hydroxides in which the metal ion has a valence of 1-3, more preferably alkali and alkaline earth metal hydroxides such as sodium hydroxide, lithium hydroxide, potassium hydroxide, calcium hydroxide, magnesium hydroxide, etc., although trivalent metal hydroxides may also be used. Ammonium hydroxide and organic hydroxides, such as tetraalkylammonium hydroxides (M is NR₄⁺ where R is C₁₋₄ alkyl or H) are also suitable for the hydrolysis reaction. The hydrolysis reaction is indicated as "Reaction 1" in Scheme 1 below in which M is a metal or ammonium cation.

In Scheme 1, the reducible substituent R is understood to be on any of rings A-D, and in any of the available positions on each ring. For example, a substituent on ring A may be present at any of the 9, 10, 11 or 12-positions. Substituents on ring B will generally be at the 7-position.

The hydroxide base is generally used in equivalent stoichiometric proportions to the camptothecin substrate. However, amounts of base slightly above or below stoichiometric proportions will obviously be effective in the hydrolysis reaction. 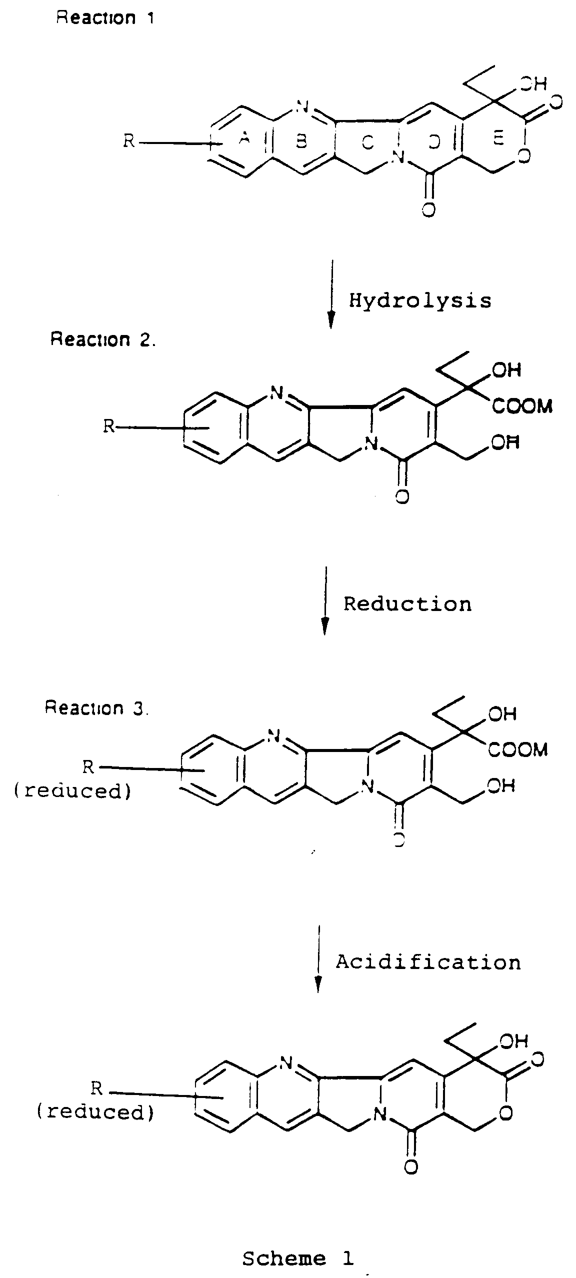
The carboxylate salt bearing the reducible substituent is then reduced to form a reduced carboxylate salt. The reduction reaction must be conducted under neutral or alkaline (pH > 7.0) conditions so that the hydrolyzed lactone does not reform the lactone E ring. The reduction can be performed in aqueous neutral or alkaline media, or organic solvents which do not interfere with the reduction reaction and in which the carboxylate salt is substantially soluble. Convenient organic solvents are protic solvents such as C₁₋₄ lower alcohols, primarily methanol, ethanol, propanol and butanol. Aprotic organic solvents such as dimethylformamide (DMF) can also be used.

Reduction reactions which may be performed include hydrogenation reactions using H₂ as a hydrogen source, such as the hydrogenation of a nitro group to an amine, an alkene to an alkane, a cyano to an aminomethyl, a halogen to a hydrogen etc., in alcohol solvent using an appropriate hydrogenation catalyst. Hydrogenation catalysts for a variety of catalytic hydrogenation reactions are well known and can be used in the reduction reaction of the present invention (c.f. H.O. House, Modern Synthetic Reactions, 2nd ed., W.A. Benjamin, Inc., Menlo Park, CA, 1972). Examples include the catalytic metals such as Pt, Pd, Ni, etc. optionally on a support such as carbon. Typically, the carboxylate salt is dissolved in absolute ethanol and hydrogenated in the presence of the hydrogenation catalyst until the hydrogenation (reduction) is substantially complete.

Reduction reactions can also be carried out in aqueous or alcohol/water media. A nitro camptothecin, such as 9-nitro-camptothecin, for example, can be smoothly reduced by the FeSO₄-NH₄OH method in ethanol/water solvent to give the corresponding amino carboxylate salt (c.f. I. Smith and J. W. Opie, Org. Synth. Collect. Vol. 3, ed. E. C. Horning, John Wiley and Sons, New York, N.Y., 1955, p.56).

In general, any conventional reduction reaction which can be conducted in alkaline or neutral media can be used as the reducing reaction in the present method. Suitable reduction reactions include not only the hydrogenation (H₂) and FeSO₄-NH₄OH methods described above, but also electrochemical reductions, and reductions using hydrazine or cyclohexene as a hydrogen source with a metal catalyst, such as Pd or Pt.

The reduced carboxylate salt can be isolated by conventional crystallization or chromatographic methods. The isolated reduced carboxylate salt is then contacted with acid, typically with an aqueous acid solution to reform the lactone E ring of the camptothecin compound as shown in Scheme 1. Alternatively, the reaction media containing the reduced carboxylate salt may be directly acidified by the addition of acid to reform the E ring. The reduced camptothecin compound having a complete lactone E ring is substantially less water soluble than the carboxylate salt and can be conveniently isolated by simple filtration and/or extraction using conventional organic solvents such as chloroform, dichloromethane, toluene, etc.

The acidification step can be conducted using a mineral acid or an organic acid. Suitable mineral acids include hydrochloric, hydrobromic acid, sulfuric acid, phosphoric acid, etc. Suitable organic acids include acetic acid, p-toluenesulfonic acid, trifluoroacetic acid, oxalic acid, tartaric acid, etc. The acid is generally added as a dilute solution, preferably 0.1-1.0 moles/liter, although the concentration of the acid is not critical.

The process of the present invention enables one to perform high yield reduction reactions on camptothecin and camptothecin analogs by converting the camptothecin to a water soluble carboxylate salt, reducing the carboxylate salt and then reforming the camptothecin skeletal structure by relactonizing the E ring. The present method results in increased yield and flexibility in synthetic procedures directed to the preparation of camptothecin analogs.

The process of the present invention may be used to synthesize 20(RS)-camptothecin, 20(S)-camptothecin or 20(R)-camptothecin derivatives or mixtures of the (R) and (S) enantiomers. When conducting reactions on a mixture of enantiomers, the formation of the carboxylate salt, the reduction reaction and reformation of the lactone ring can all be performed on the mixture. The resulting mixture of reduced (R) and (S) compounds, can be conveniently resolved into separate enantiomers using known resolution processes. For example, 20(RS)-camptothecin derivatives can be resolved by reaction with a suitable chiral amine, such as R-(+)-α-methyl benzylamine or S-(-)-α-methyl benzylamine to form the corresponding diastereomeric amides by the method disclosed in U.S. application serial no. 07/511,953 incorporated herein by reference. After separation of the diastereomeric amides, the amides are treated with acid to reform the complete camptothecin ring structure.

Other features of the invention will become apparent in the course of the following descriptions of exemplary embodiments which are given for illustration of the invention and are intended to be limiting thereof. In the following examples, examples actually reduced to practice are written in the past tense, examples constructively reduced to practice are written in the present tense.

### EXAMPLES

### Example 1 - Preparation of 10-amino-20(RS)-camptothecin by the FeSO₄ -NH₄OH Method

A suspension of 10-nitro-20(RS)-camptothecin (200 mg, 0.5 mmol) in 90% MeOH (50 ml) and 0.1N NaOH (5 ml) was heated at 50-60°C for 1 hr. under nitrogen. Most of the MeOH was evaporated under reduced pressure, the brown solid obtained was redissolved in water (10 ml) and filtered through a 0.45 micron filter membrane to remove any insoluble material. The water was removed under reduced pressure to give the sodium salt of 10-nitro-20(RS)-camptothecin (213 mg, yield 97%).

The sodium salt of 10-nitro-20(RS)-camptothecin (200 mg, 0.46 mmol) was dissolved in hot 50% aqueous ethanol (20 ml) and added to a solution of FeSO₄.7H₂0 (2.0 g, 7.2 mmol) in boiling water (20 ml). Ammonium hydroxide (3 ml) was added slowly to the boiling solution. The reaction mixture was filtered hot through a "celite" pad washed with hot water (10 ml). The yellow fluorescent filtrate was neutralized with dilute HC1 and was heated on a steam bath for 15 minutes. The filtrate was then cooled and extracted with CHC1₃ (5 x 100 ml). The dried (Na₂SO₄) chloroform extract was evaporated under reduced pressure to give pure 10-amino-20(RS)-camptothecin (184 mg, 100%). The spectral properties of this sample were identical with those of an authentic sample.

### Example 2 - Preparation of 10-amino-20(RS)-camptothecin by a Catalytic Reduction Method

A mixture of the sodium salt of 10-nitro-20(RS)-camptothecin (11.0 mg, 0.025 mmol) prepared as in Example 1, and 10% Pd/C (6.0 mg) in absolute EtOH (10 ml) was subjected to 1 atm. of H₂ for 1 hour. The catalyst was removed by filtration through "celite". Additional absorbed product was obtained by a thorough washing of the filter cake with absolute EtOH (50 ml). The filtrate was acidified with dilute HC1, warmed on a water bath for 10 minutes and extracted with CHC1₃ (5 x 25 ml). The dried (Na₂SO₄) chloroform extract was evaporated under reduced pressure to give 10-amino-20(RS)-camptothecin (7.0 mg, 76%). The spectral properties of this sample were identical with those of an authentic sample.

### Example 3 - Preparation of 9-amino-20(RS)-camptothecin by the FeSO₄-NH₄OH Method

A suspension of 9-nitro-20(RS)-camptothecin (200 mg, 0.5 mmol) in 90% MeOH (50 ml) and 0.1 N NaOH (5 ml) was heated at 50-60° C for 1 hour under nitrogen. Most of the MeOH was evaporated under reduced pressure, the brown solid obtained redissolved in water (10 ml), and filtered through a 0.45 micron filter membrane to remove any insoluble impurities. The solvent was removed under reduced pressure to give the sodium salt of 9-nitro-20(RS)-camptothecin (215 mg, 98%).

The sodium salt of 9-nitro-20(RS)-camptothecin (213 mg, 0.51 mmol) was dissolved in hot 50% aqueous ethanol (20 ml) and added to a solution of FeSO₄·7H₂O (2.0 g, 7.2 mmol) in boiling water (20 ml). Ammonium hydroxide (3 ml) was added slowly to the boiling solution. The reaction mixture was filtered hot through a "celite" pad and washed with hot water (10 ml). The filtrate was acidified with dilute HC1 and was heated on a steam bath for 15 minutes. The yellow precipitate was filtered and dried (137 mg). The filtrate was extracted with CHC1₃ (5 x 50 ml). The dried (Na₂SO₄) chloroform extract was evaporated under reduced pressure to give pure 9-amino-20(RS) camptothecin. The total pure product weighed 146.0 mg (81%). The spectral properties of this sample were identical with those of an authentic sample.

### Example 4 - Preparation of 9-amino-20(RS)-camptothecin by a Catalytic Reduction Method

A mixture of the sodium salt of 9-nitro-20(RS)-camptothecin (20.0 mg, 0.46 mmol), prepared as in Example 3, and 10% Pd/C (10.0 mg) in absolute EtOH (5 ml) was subjected to 1 atm. of H₂ for 18 hours. The catalyst was removed by filtration through "celite". Additional absorbed product was obtained by a thorough washing of the filter cake with absolute EtOH (50 ml). The filtrate was acidified with dilute HC1 and warmed on a water bath for 10 minutes. The brown precipitate of 9-amino-20(RS) camptothecin (6.2 mg) was filtered. The filtrate was extracted with CHC1₃ (5 x 20 ml). The dried (Na₂SO₄) chloroform extract was evaporated under reduced pressure to give an additional amount of 9-amino-20(RS)-camptothecin (3.6 mg). The total yield of the product was 9.8 mg (58%). The spectral properties of this sample were identical with those of an authentic sample.

### Example 5

By a procedure analogous to Examples 1 and 2, 11-nitro-20(RS)-, 9-nitro-20(R)-, 9-nitro-20(S)-, 10-nitro-20(R)-, 10-nitro-20(S)-, 11-nitro-20(R)-, and 11-nitro-20(S)-camptothecin compounds are reduced by the FeSO₄-NH₄OH or catalytic reduction to the corresponding 11-amino-20(RS)-, 9-amino-20(R)-, 9-amino-20(S)-, 10-amino-20(R)-, 10-amino-20(S)-, 11-amino-20(R)- and 11-amino-20(S)-camptothecin derivatives.

### Example 6

Analogous to Example 1, a suspension of 9-nitro-10,11-methylenedioxy-20(S)-camptothecin in methanol containing NaOH is heated to obtain the 9-nitro-10,11-methylenedioxy-20(S)-camptothecin sodium salt. The sodium salt is then dissolved in hot 50% aqueous ethanol and added to a solution of FeSO₄.7H₂O in boiling water. Ammonium hydroxide is added to the boiling solution in the manner described in Example 1. After filtration, acidification and work up analogous to Example 1, 9-amino-10,11-methylenedioxy-20(S)-camptothecin is obtained.

Obviously, numerous modifications and variations of the present invention are possible in light of the above teachings. It is therefore to be understood that within the scope of the appended claims, the invention may be practiced otherwise than as specifically described herein.

## Claims

1. A process for preparing a reduced camptothecin, comprising the steps of:
(1) hydrolyzing under alkaline conditions the lactone E ring of a camptothecin compound having the structure 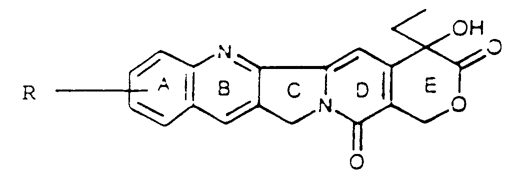 wherein at least one of the A, B, C or D rings is substituted with at least one substituent R which is capable of being reduced under alkaline or neutral conditions, to form a water soluble substituted carboxylate of the formula 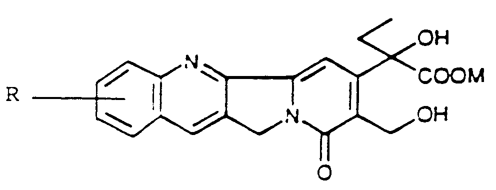 wherein M is a cation;
(2) reducing said substituent R in alkaline or neutral media to form a reduced carboxylate; and
(3) contacting said reduced carboxylate with an acid to reform said lactone E ring.

2. The process of Claim 1, wherein M is a monovalent, divalent or trivalent metal cation.

3. The process of Claim 1, wherein M is an alkali or alkaline earth metal cation, a tetra-C₁₋₄-alkyl-ammonium or an ammonium ion.

4. The process of Claim 1, wherein said acid is a mineral acid.

5. The process of Claim 4, wherein said mineral acid is hydrochloric, hydrobromic, sulfuric or phosphoric acid.

6. The process of Claim 1, wherein said substituent R is selected from the group consisting of nitro, cyano, azido, alkenyl, alkynyl, halide, nitroso and formyl groups.

7. The process of Claim 6, wherein said substituent is a nitro group in the 9, 10, 11 or 12 position of the A ring.

8. The process of Claim 1, wherein said reducing reaction is a catalytic hydrogenation.

9. The process of Claim 1, wherein said reducing reaction is a reduction with FeSO₄-NH₄OH.

10. The process of Claim 8, wherein said catalytic hydrogenation is conducted using H₂ as a hydrogen source.

11. The process of Claim 8, wherein said catalytic hydrogenation is conducted using hydrazine as a hydrogen source.

12. The process of Claim 8, wherein said catalytic hydrogenation is conducted using cyclohexene as a hydrogen source.

13. The process of Claim 1, where said reducing reaction is an electrochemical reduction.
